# EUROPÄISCHE PATENTSCHRIFT

(11) **EP 2 759 255 B1**
(45) Veröffentlichungstag und Bekanntmachung des Hinweises auf die Patenterteilung: **16.08.2017**
(21) Anmeldenummer: 13197769.6
(22) Anmeldetag: 17.12.2013
(51) Int. Cl.: A61B 5/00

(54) **Zum Durchführen optischer Absorptionsspektroskopie ausgestaltete Anordnung und Vorrichtungen**
Assembly for performing optical absorption spectroscopy and devices
Système et dispositifs permettant de réaliser une spectroscopie par absorption optique

(30) Priorität: 28.01.2013 DE 102013201275
(43) Veröffentlichungstag der Anmeldung: 30.07.2014
(73) Patentinhaber: ROBERT BOSCH GMBH, 70442 Stuttgart (DE)
(72) Erfinder: Ramsteiner, Ingo, 71229 Leonberg (DE)

(56) Entgegenhaltungen:
- WO-A1-01/26540
- DE-A1-102010 001 220
- US-A- 5 143 066
- US-A1- 2011 224 514
- US-B1- 6 256 522

## Beschreibung

Die Erfindung betrifft eine Anordnung mit Vorrichtungen, die das Durchführen einer optischen Absorptionsspektroskopie erlauben.

### Stand der Technik

Das Absorptionsspektrum von lebendem Gewebe, insbesondere Haut, weist über weite Bereiche relativ geringe Lichtabsorption auf und ist damit grundsätzlich der Spektroskopie mit entsprechenden Lichtwellenlägen zugänglich. Eine technische Schwierigkeit ist dabei die starke Streuung des Lichts im Gewebe. Das Licht kann je nach Wellenlänge zentimeterweise Strecken im Gewebe zurücklegen, legt diese Strecken jedoch nur über unzählige Streuprozesse hinweg. Die Ausbreitung des Lichts im Gewebe entspricht vielmehr einem Diffusionsprozess als einer Lichtausbreitung über geradlinigen Strahlen. Durch diese Unbestimmtheit der Lichtwege im Gewebe, wird das Durchführen der Spektroskopie im Gewebe erschwert. Die daraus resultierenden Ergebnisse können dabei verfälscht werden. Ferner kann es vorkommen, dass schwach absorbierende Teilchen (z.B. Moleküle, Ionen, Elektronen) als Analyten in kleinsten Konzentrationen schwer bis kaum zu quantifizieren sind. Ein solches schwach absorbierendes Molekül ist beispielsweise die Glukose.

Daher besteht nach wie vor Bedarf nach verbesserten Vorgehensweisen und Vorrichtungen zum Durchführen einer optischen Absorptionsspektroskopie. Dabei ist es beispielsweise wünschenswert, trotz der starken Streuung des Lichts im Gewebe, verschiedene Analyten unabhängig von ihrer Absorptionsstärke zuverlässig zu quantifizieren.

Aus den Dokumenten US 6,256,522 B1 und US 2011/0224514 A1 sind unter die Haut implantierbare Vorrichtungen zur Messung von Eigenschaften von körpereigenen Analyten offenbart.

### Offenbarung der Erfindung

Die vorliegende Erfindung wird im Wesentlichen durch Merkmale der unabhängigen Ansprüche angegeben. Weitere Ausgestaltungen der vorliegenden Erfindung, können beispielhaft den abhängigen Ansprüchen entnommen werden.

Die Idee der vorliegenden Erfindung besteht darin, die zum Durchführen der Absorptionsspektroskopie notwendige Lichtquelle wie ein Implantat unter die Hautoberfläche zu platzieren. Eine solche Lichtquelle sollte möglichst punkförmig sein und das Licht nur in die Richtung des Detektors, der das Licht der Lichtquelle detektiert, ausstrahlen. Dabei setzt die vorliegende Erfindung die Lichtquelle als einen Fluoreszenzkonzentrator um. Dabei wird ein Fluoreszenzfarbstoff auf eine transparente Platte aufgebracht oder in das Material der Platte eingebettet. Durch die Hautoberfläche einfallendes Licht regt den Farbstoff zu isotrop abgestrahlter Fluoreszenz an. Da die Abstrahlung innerhalb des Materials erfolgt, bleibt ein Großteil des Lichts über Totalreflexion in der Platte gefangen wie in einem Lichtleiter und kann an einer dafür vorgesehenen Stelle möglichst punktuell ausgekoppelt werden. Dadurch, dass die erfindungsgemäße Lichtquelle wie ein Fluoreszenzkonzentrator umgesetzt ist, kann die Lichtquelle diffuses Licht, wie es auch im Gewebe nach der Ausstrahlung durch die Hautoberfläche vorliegt, konzentrieren. Der Fluoreszenzkonzentrator fängt das ins Gewebe einfallende Licht mit seiner ganzen Fläche auf, wandelt es in ein Fluoreszenzlicht größerer Wellenlänge und strahlt dieses von annähernd einem Punkt aus in die Richtung des Detektors an der Hautoberfläche ab. Dort nachgewiesen, kann das ausgestrahlte Fluoreszenzlicht mit den bekannten Methoden der Absorptionsspektroskopie ausgewertet werden.

Durch die vorliegende Erfindung können, trotz der starken Streuung des Lichts im Gewebe, verschiedene Analyten unabhängig von ihrer Absorptionsstärke zuverlässig quantifiziert werden. Ferner sind die Ergebnisse der Absorptionsspektroskopie zuverlässig, da die vorliegende Erfindung das im Gewebe diffus gestreute Licht gut einsammeln und konzentrieren kann und das für die Absorptionsspektroskopie notwendige Licht mit größeren Wellenlängen in die Richtung der Hautoberfläche strahlen kann. Die Wellenlängen des durch die erfindungsgemäße Lichtquelle emittierten Lichts sind größer als die des durch die erfindungsgemäße Lichtquelle absorbierten Lichts. Ferner ist zum Durchführen der Absorptionsspektroskopie seitens der Lichtquelle keine Energieversorgung notwendig.

In den folgenden Figuren wird die Erfindung anhand von Ausführungsformen schematisch genauer beschrieben. Dabei können zur Übersichtlichkeit gleiche oder gleichwirkende Elemente mit gleichen Bezugszeichen versehen sein.
Fig. 1 zeigt eine Anordnung gemäß einer Ausführungsform der vorliegenden Erfindung;
Fig. 2 zeigt eine Licht detektierende Vorrichtung gemäß einer Ausführungsform der vorliegenden Erfindung;
Fig. 3 zeigt die spektralen Verhältnisse zwischen dem durch die fluoreszierende Schicht absorbierten Licht und dem abgestrahlten oder emittierten Fluoreszenzlicht gemäß einer Ausführungsform der vorliegenden Erfindung;
Fig. 4 zeigt die spektralen Verhältnisse zwischen anderweitig rückgestrahlten Licht, das nach der Bestrahlung mit dem Licht einer Licht detektierenden Vorrichtung rückgestrahlt wurde, und dem emittierten Fluoreszenzlicht gemäß einer Ausführungsform der vorliegenden Erfindung;
Fig. 5 zeigt eine Ausgestaltung der unter die Hautoberfläche einsetzbaren Vorrichtung gemäß einer Ausführungsform der vorliegenden Erfindung;
Fig. 6 zeigt eine Anordnung gemäß einer Ausführungsform der vorliegenden Erfindung; und
Fig. 7 zeigt eine Ausgestaltung der unter die Hautoberfläche einsetzbaren Vorrichtung gemäß einer Ausführungsform der vorliegenden Erfindung.

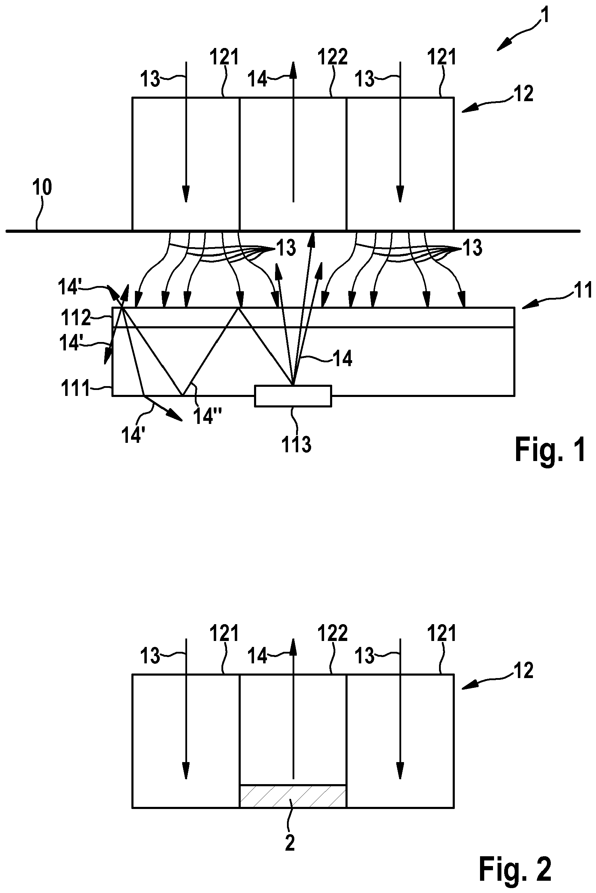

Fig. 1 zeigt eine Anordnung 1 gemäß einer Ausführungsform der vorliegenden Erfindung. Die Anordnung 1 weist gemäß der vorliegenden Ausführungsform eine Vorrichtung 11 (als die oben erwähnte Lichtquelle) auf, die: ausgestaltet ist, unter eine Hautoberfläche 10 eingesetzt zu werden; eine fluoreszierende Schicht 112 aufweist, wobei die fluoreszierende Schicht 112 ausgestaltet ist, ein auf die Hautoberfläche 10 abgestrahltes Licht 13 zu absorbieren und das absorbierte Licht in ein Fluoreszenzlicht zu wandeln; einen das Fluoreszenzlicht abstrahlenden Bereich 113 aufweist, der ausgestaltet ist, das Fluoreszenzlicht aufzunehmen und in die Richtung der Hautoberfläche 10 abzustrahlen oder zu emittieren (siehe die Pfeile mit dem Bezugszeichen 14). Die fluoreszierende Schicht 112 kann beispielsweise auf einer der Hautoberfläche 10 zugewandten Seite der Vorrichtung 111 ausgebildet sein. Gemäß der vorliegenden Ausführungsform weist die Vorrichtung 11 ferner ein Substrat 111 auf. Die fluoreszierende Schicht 112 kann beispielsweise auf dem Substrat 111 ausgebildet sein. Im Substrat 111 das Fluoreszenzlicht aufgenommen (siehe Bezugszeichen 14") und dem das Fluoreszenzlicht abstrahlenden Bereich 113 zugeleitet. Der Bereich 113, der das Fluoreszenzlicht 14", 14 abstrahlt, koppelt das in der Vorrichtung 11 aufgenommene Fluoreszenzlicht 14", 14 aus und emittiert es in die Richtung der Hautoberfläche 11. Da die Vorrichtung 11 unter die Hautoberfläche 10 eingesetzt wird, kann sie auch als Implantat bezeichnet werden. Durch die Vorrichtung 11 wird das Untersuchen des Gewebes, der Flüssigkeiten und/oder der Teilchen, die sich zwischen der Vorrichtung 11 und der Hautoberfläche 10 befinden, ermöglicht und verbessert. Wie bereits erwähnt, besteht und entsteht bei den Untersuchungen mittels Licht oft das Problem der starken Streuung des Lichts unter der Hautoberfläche 10, was die Spektroskopie des Gewebes, der Flüssigkeiten und/oder der Teilchen erschwert. Durch die vorliegende Erfindung wird ein großer Anteil des unter der Hautoberfläche 10 gestreuten Lichts durch die Vorrichtung 11 aufgenommen oder absorbiert, so dass der Anteil des anderweitig unter der Hautoberfläche 10 gestreuten Lichts geringfügig bleibt und das Ergebnis der Spektroskopie nicht mehr nachteilig beeinflussen kann. Über die Totalreflexion in der Vorrichtung 11 (z.B. in ihrer Platte oder in dem Substrat 111) bleibt das absorbierte Licht als Fluoreszenzlicht in der Vorrichtung 11 wie in einem Lichtleiter gefangen und wird nur in der dafür vorgesehenen Stelle der Vorrichtung 11 - in dem das Fluoreszenzlicht abstrahlenden Bereich 113 - ausgekoppelt und in die Richtung der Hautoberfläche 10 abgestrahlt. Die vorliegende Erfindung gestaltet die Vorrichtung 11 als einen Fluoreszenzkonzentrator aus, der diffuses Licht konzentrieren kann. Das durch die Vorrichtung 11 (d.h. durch den das Fluoreszenzlicht abstrahlenden Bereich 113) in die Richtung der Hautoberfläche 10 abgestrahlte Fluoreszenzlicht kann gut von dem anderweitigen durch die Hautoberfläche 10 abgestrahlten Licht 14' unterschieden werden, was die Zuverlässigkeit der Ergebnisse der Spektroskopie deutlich erhöht und verbessert. Das anderweitige durch die Hautoberfläche 10 abgestrahlte Licht 14' kann von der Vorrichtung 11 anderweitig, d.h. nicht durch den Bereich 113 abgestrahltes Licht sein und/oder sonst vom Gewebe emittiertes Licht umfassen. Für das Auswerten des durch die Vorrichtung 11 abgestrahlten Fluoreszenzlichts können beispielsweise verschiedene bekannte Methoden der Absorptionsspektroskopie verwendet werden. Ferner wird durch die vorliegende Erfindung keine Stromversorgung für die Vorrichtung 11 benötigt. Die Vorrichtung 11 kann darüber hinaus (als Implantat) grundsätzlich unbegrenzte Zeit im Körper, bzw. unter der Hautoberfläche 10 verbleiben, wodurch die Untersuchungen des Gewebes, der Flüssigkeiten und/oder der Teilchen jederzeit wiederholt werden können und wodurch die Untersuchungen auf eine einfache Weise und ohne weitere Eingriffe vorgenommen werden können.

Zum Bestrahlen der Hautoberfläche 10 und somit zum Zuführen des Lichts 13 zu der Vorrichtung 11 durch die Hautoberfläche 10 weist die Anordnung 1 gemäß der vorliegenden Ausführungsform eine Licht detektierende Vorrichtung 12 auf. Gemäß der vorliegenden Ausführungsform ist die Licht detektierende Vorrichtung 12 ausgestaltet, Licht 13 auf die Hautoberfläche 10 abzustrahlen und das Fluoreszenzlicht 14, das von der Vorrichtung 11 erfindungsgemäß abgestrahlt oder emittiert wurde, zu empfangen. Dabei wird das von der Vorrichtung 11 erfindungsgemäß emittierte Fluoreszenzlicht 14 von der Licht detektierenden Vorrichtung 12 zum Auswerten des emittierten Fluoreszenzlichts 14 verwendet. Die Vorrichtung 11 strahlt das Fluoreszenzlicht 14 erst nach dem Abstrahlen des Lichts 13 auf die Hautoberfläche 10 ab. Gemäß der vorliegenden Ausführungsform geschieht dieses somit in Erwiderung auf das Abstrahlen des Lichts 13 durch die Licht detektierende Vorrichtung 12.

Gemäß der vorliegenden Ausführungsform weist die Licht detektierende Vorrichtung 12 zumindest ein Licht abstrahlendes Element 121 auf, das ausgestaltet ist, Licht 13 auf die Hautoberfläche 10 abzustrahlen. Ferner weist die Licht detektierende Vorrichtung 12 zumindest ein Licht empfangendes Element 122 auf, das ausgestaltet ist, das Fluoreszenzlicht 14, das von der Vorrichtung 11 (d.h. ihren dafür vorgesehenen Bereich 113) abgestrahlt oder emittiert wurde, zu detektieren und zu empfangen. Die Licht detektierende Vorrichtung 12 kann nach dem Empfangen des Fluoreszenzlichts 14 dieses auswerten oder einer weiteren dafür vorgesehenen Vorrichtung zum Auswerten bereitstellen. Die Licht detektierende Vorrichtung 12 kann beispielsweise eine Sonde sein. Das zumindest eine Licht abstrahlende Element 121 kann beispielsweise eine Glasfaser sein, die zum Beleuchten ausgestaltet ist. Das zumindest eine Licht empfangende Element 122 kann beispielsweise eine Glasfaser sein, die zum Nachweis des empfangenen Fluoreszenzlicht 14 ausgestaltet ist.

Fig. 1 zeigt die Vorrichtung 11 in einem unter die Hautoberfläche 10 eingesetzten oder implantierten Zustand. Die genaue Tiefe bzw. die Dicke der Hautschicht richtet sich je nach Anwendungsbereich der Anordnung 1 mit den beiden Vorrichtungen 11 und 12. Dabei befindet sich das spektroskopisch zu untersuchende Gewebe zwischen der Vorrichtung 11 (z.B. dem Implantat 11) und der auf die Hautoberfläche 10 aufgesetzten Licht detektierenden Vorrichtung 12 (z.B. einer Sonde 12). Über die Licht detektierende Vorrichtung 12 wird Licht 13 (im Nachfolgenden auch als Anregungslicht bezeichnet) in die Haut 10 eingestrahlt, das sich diffus im Gewebe ausbreitet. Die Vorrichtung 11 sammelt (durch die fluoreszierende Schicht 112) einen großen Teil des in die Haut 10 eingestrahlten Lichts 13 ein, wandelt es (durch die fluoreszierende Schicht) in das Fluoreszenzlicht 14' und strahlt das erhaltene Fluoreszenzlicht 14', 14 in die Richtung der Licht detektierenden Vorrichtung 12 zurück.

Die Vorrichtung 11 kann flächig ausgestaltet sein, wodurch die Vorrichtung 11 eine verbesserte Eigenschaft der Lichtabsorbation erhält, d.h. die Vorrichtung 11 kann mehr von dem auf die Hautoberfläche 10 ausgestrahlten und im Gewebe verteilten Licht 13 aufsammeln bzw. absorbieren.

Der das Fluoreszenzlicht abstrahlende Bereich 113 kann kleiner als die der Hautoberfläche zugewandte Seite der Vorrichtung 11 sein. In diesem Fall ist der das Fluoreszenzlicht abstrahlende Bereich 113 kleiner als die fluoreszierende Schicht 112. Dadurch wird eine bessere Unterscheidbarkeit des durch die Vorrichtung 11 (bzw. durch den Bereich 113) erfindungsgemäß emittierten Fluoreszenzlichts 14 von dem anderweitig durch die Hautoberfläche 10 emittierten Licht 14' ermöglicht. Der das Fluoreszenzlicht abstrahlende Bereich 113 kann dabei derart ausgestaltet sein, dass er aus der Sicht der Licht detektierenden Vorrichtung 12 eine nahezu punktförmige, im Gewebe untergebrachte Lichtquelle darstellt, die zur Absorptionsspektroskopie des zwischen den beiden Vorrichtungen 11 und 12 liegenden Gewebes, der zwischen den beiden Vorrichtungen 11 und 12 liegenden Flüssigkeiten und/oder der zwischen den beiden Vorrichtungen 11 und 12 liegenden Teilchen genutzt werden kann. Auf diese Weise wird das in der Vorrichtung 11 gefangene Licht 14' durch den Bereich 113 punktuell ausgekoppelt und emittiert (siehe Pfeile 14). Die Absorptionsspektroskopie des erfindungsgemäß emittierten Fluoreszenzlichts 14 kann dann durch die Licht detektierende Vorrichtung 12 oder eine anderweitige geeignete Vorrichtung mittels dafür bekannter Verfahren vorgenommen werden.

Gemäß der vorliegenden Ausführungsform ist die Wellenlänge des (durch den das Fluoreszenzlicht abstrahlenden Bereich 113) abgestrahlten oder emittierten Fluoreszenzlichts 14 größer als die Wellenlänge des (durch die fluoreszierende Schicht 112) absorbierten Lichts 13. Dabei überlappen sich die Absorption- und Emmissionsbanden des für die fluoreszierende Schicht 112 verwendeten Fluoreszenzmittels spektral nur teilweise und vorzugsweise überhaupt nicht. Auf diese Weise lassen sich das von der Vorrichtung 11 erfindungsgemäß abgestrahlte Fluoreszenzlicht 14 und das anderweitig vom Gewebe und von der Vorrichtung 11 rückgestreute Licht 14' mittels bekannter Verfahren der Spektroskopie gut unterscheiden. Die Licht detektierende Vorrichtung 12 kann ein Filterelement 2 aufweisen, welches das von der Vorrichtung 11 abgestrahlte Fluoreszenzlicht 14 zum Auswerten durch die Licht detektierende Vorrichtung 12 durchlässt und ein anderweitiges von der Hautoberfläche 10 emittiertes Licht 14' blockiert. Eine beispielhafte Ausführungsform der Licht detektierenden Vorrichtung 12 mit dem Filterelement 2 ist in Fig. 2 gezeigt. Das Filterelement 2 kann auf der zu der Hautoberfläche 10 ausgerichteten Seite der Licht detektierenden Vorrichtung 12 vor dem Licht empfangenden Element 122 eingerichtet sein. Das Filterelement 2 kann beispielsweise ein spektraler Kantenfilter sein, der Licht mit Wellenlängen unterhalb der Fluoreszenzbande der Vorrichtung 11 blockiert. Durch das Verwenden des Filterelements 2, welches durch die oben erläuterte Unterscheidbarkeit des von der Vorrichtung 11 (bzw. durch den Bereich 113) emittierten Fluoreszenzlichts 14 ermöglicht wird, wird sichergestellt, dass nur das tatsächlich zu untersuchende Licht bei der Auswertung mittels Spektroskopie, insbesondere mittels Absorptionsspektroskopie, herangezogen wird. Somit wird die Aussagekraft, Sicherheit, Zuverlässigkeit der ausgewerteten Werte erhöht. Insgesamt wird das Untersuchen des Gewebes, der Flüssigkeiten und/oder der Teilchen zwischen der Hautoberfläche 10 und der Vorrichtung 11 präziser und einfacher.

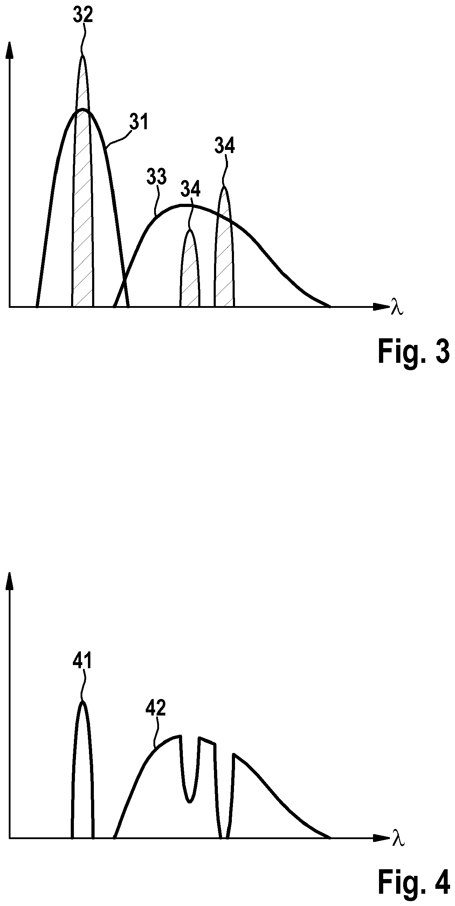

Fig. 3 zeigt die spektralen Verhältnisse zwischen dem durch die fluoreszierende Schicht 112 absorbierten Licht und dem abgestrahlten oder emittierten Fluoreszenzlicht 14 gemäß einer Ausführungsform der vorliegenden Erfindung. Die horizontale Achse gibt dabei die Wellenlänge λ in nm an und die vertikale Achse gibt dabei die Stärke der Absorption oder der Emission an, wobei auf der horizontalen Achse die Wellenlänge λ (von links nach rechts) aufsteigend angegeben wird. Gemäß Fig. 2 absorbiert die fluoreszierende Schicht 112 das durch die Hautoberfläche 10 durchstrahlende Licht 13 in einem Bereich 31, der den Bereich 32 des durch die Licht detektierende Vorrichtung 12 ausgestrahlten Lichts 13 abdeckt. Das Fluoreszenzlicht 14 wird gemäß der vorliegenden Ausführungsform in einem Bereich 33 emittiert oder abgestrahlt, der von dem Absorptionsbereich 31 verschieden oder getrennt ist und der die charakteristischen Absorptionsbanden 34 des Analyten (d.h. des zu untersuchenden Gewebes, der zu untersuchenden Flüssigkeit und/oder der zu untersuchenden Teilchen) umfasst.

Fig. 4 zeigt die spektralen Verhältnisse zwischen dem anderweitig rückgestrahlten Licht (z.B. 14' in Fig. 1), das nach der Bestrahlung mit dem Licht 13 der Licht detektierenden Vorrichtung 12 rückgestrahlt wurde, und dem durch die Vorrichtung 11 (bzw. durch den Bereich 113) emittierten Fluoreszenzlicht 14 gemäß einer Ausführungsform der vorliegenden Erfindung. Das emittierte Fluoreszenzlicht 14 ist dabei durch die Absorption des Analyten (d.h. des zu untersuchenden Gewebes, der zu untersuchenden Flüssigkeit und/oder der zu untersuchenden Teilchen) lokal abgeschwächt. In Fig. 4 gehört der Bereich 41 gehört zu dem anderweitigen rückgestrahlten Licht (z.B. 14' in Fig. 1) und der Bereich 42 gehört zu dem emittierten Fluoreszenzlicht 14, welches durch die Absorption des Analyten lokal abgeschwächt ist. Im Bereich 42 ist das Fluoreszenzsignal mit Absorptionslinien des Analyten dargestellt. Die Achsen der Fig. 4 entsprechen den Achsen der Fig. 3.

Gemäß einer Ausführungsform der vorliegenden Erfindung besteht die Vorrichtung 11 oder das Substrat 111 der Vorrichtung 11 aus einem hochbrechenden (high-n) Material. Vorzugsweise ist der Brechungsindex des Materials der Vorrichtung 11 oder des Substrats 111 größer als der Brechungsindex des umgebenden Biomaterials oder des umgebenden Gewebes respektive. Dadurch wird die Unterscheidbarkeit zwischen dem von der Vorrichtung 11 abgestrahlten oder emittierten Fluoreszenzlicht 14 und dem sonstigen über die Hautoberfläche 10 abgestrahlten oder emittierten Licht 14' unterstützt, da die Totalreflexion durch an den Grenzflächen zu höher brechenden Medien auftritt, d.h. hier an den Grenzflächen vom Gewebe zu der Vorrichtung 11 oder zu dem Substrat 111 der Vorrichtung 11 respektive. Auf diese Weise wird die Zuverlässigkeit der Ergebnisse der Spektroskopie und insbesondere der Absorptionsspektroskopie beim Untersuchen des emittierten Fluoreszenzlichts 14 erhöht. Wenn beispielsweise für die Gewebe-Flüssigkeit der Brechungsindex n als n = 1,36 gegeben ist, könnte das für die Vorrichtung 11 oder für das Substrat 111 beispielsweise bei den folgenden verwendeten Materialien die folgenden Brechungsindizes haben: bei Glas den Brechungsindex n = 1,52, bei Kunststoff wie z.B. Polymethyl-Methacrylat (PMMA) den Brechungsindex n = 1,49, bei Kunststoff wie z.B. Polystyrol den Brechungsindex n = 1,58. Dabei ist anzumerken, dass die obigen Angaben der Materialien und der dazugehörigen Brechungsindizes lediglich Beispiele sind und dass die vorliegende Erfindung das Verwenden anderer geeigneter Materialien und/oder anderer geeigneter Brechungsindizes erlaubt. Grundsätzlich sollte die Brechzahl der Vorrichtung 11 oder des Substrats 111 möglichst groß sein, auf jeden Fall größer als die Brechzahl des die Vorrichtung 11 umgebenden biologischen Materials (des Gewebes).

Gemäß einer Ausführungsform der vorliegenden Erfindung weist die fluoreszierende Schicht 112 einen Brechungsindex auf, der mit dem Brechungsindex der Vorrichtung 11 oder des Substrats 111 vergleichbar ist, z.B. gleich oder annähernd gleich ist. Dadurch werden Fresnel-Reflektionen an der Grenzfläche zwischen den Materialien der Schicht 112 und des Substrats 111 minimiert und die ungehinderte Leitung des Lichts 14" zur Auskoppelstelle 113 sichergestellt.

Die fluoreszierende Schicht 112 kann eine der wie folgt ausgestalteten Schichten sein: eine Schicht aus einem fluoreszierenden Farbstoff oder eine Schicht, die aus dem Material des Substrats 111 oder der Vorrichtung 11 und aus einem fluoreszierenden Farbstoff ausgestaltet ist, wobei der fluoreszierende Farbstoff in das Material des Substrats 111 oder der Vorrichtung 11 eingebettet ist. Die vorliegende Erfindung erlaubt zur Ausgestaltung der fluoreszierenden Schicht 112 eine gewisse Flexibilität. Die oben beschriebenen möglichen Ausgestaltungen der fluoreszierenden Schicht 112 unterstützen die Eigenschaft der Unterscheidbarkeit des durch die Vorrichtung 11 (bzw. durch den das Fluoreszenzlicht abstrahlenden Bereich 113) emittierten Fluoreszenzlichts 14 und des anderweitig emittierten Lichts 14'. Ferner wird die Zuverlässigkeit der nach der Auswertung des emittierten Fluoreszenzlichts 14 erhaltenen Ergebnisse erhöht. Wenn zum Beispiel das Material des Substrats 111 oder der Vorrichtung 11 PMMA ist, dann kann das PMMA zum Ausbilden der fluoreszierenden Schicht 112 mit einem fluoreszierenden Farbstoff eingefärbt werden. In einem solchen Fall ist eine Trennung von Substrat 111 und fluoreszierender Schicht 112 nicht mehr zwingend erforderlich - statt dessen kann das Substrat 111 auch homogen mit dem Fluoreszenzfarbstoff eingefärbt sein und auf eine vom Substrat unterscheidbare fluoreszierende Schicht 112 ganz verzichtet werden. Somit erlaubt die vorliegende Erfindung eine Ausführungsform mit einer Schicht (mit 111 und 112), in der das Substrat 111 und die fluoreszierende Schicht 112 durch Einfärben oder Einbringen des Fluoreszenzfarbstoffs im Material des Substrats 111 verbunden oder vereint. Die vorliegende Beschreibung behandelt beide Schichten - das Substrat 111 und die fluoreszierende Schicht 112 - zwecks klarer und kurzer Darstellung zwar getrennt, umfasst jedoch auch die Ausführungsform mit nur einer, die Schichten 111 und 112 verbindenden oder vereinenden Schicht. Dabei sind die Merkmale, Vorteile, Funktionen und weiteren Ausgestaltungen des Substrats 111 und der fluoreszierenden Schicht 112 auch durch die eine beide Schichten 111, 112 umfassende Schicht entsprechend umfasst.

Die Absorptions- und Emissionsbanden des Fluoreszenzmittels oder des Fluoreszenzfarbstoffs überlappen sich spektral nur teilweise oder gar nicht, wodurch ebenfalls die oben genannten Eigenschaften der Unterscheidbarkeit des emittierten Fluoreszenzlichts 14 von dem anderweitig emittierten Licht 14' und der Zuverlässigkeit der Ergebnisse der Auswertung des emittierten Fluoreszenzlichts 14 gewährleistet und unterstützt werden. So kann das Fluoreszenzmittel oder der Fluoreszenzfarbstoff das auf die Hautoberfläche abgestrahlte Licht 13 nur in einem begrenzten Spektralbereich absorbieren und mit hoher Quanteneffizienz (z.B. größer als 90%) in einem ebenfalls begrenzten, von der Absorptionsbande getrennten Spektralbereich durch Fluoreszenz abgeben oder emittieren. Das Fluoreszenzmittel oder der Fluoreszenzfarbstoff kann beispielsweise ein Laserfarbstoff, eine Neonfarbe (z.B. für Textmarker) oder auf organische Farbstoffe wie z.B. Perylen oder Naphtalimid basierter Stoff sein. Alternativ können zum Ausbilden der fluoreszierenden Schicht Quantenpunkte eingesetzt werden.

Generell sollte die Auswahl des Fluoreszenzmittels oder der Fluoreszenzfarbstoffs in Abstimmung mit der jeweiligen Anwendung erfolgen. Um die positiven Effekte der vorliegenden Erfindung zu unterstützen und zu gewährleisten, sollte die Absorptionsbande des Fluoreszenzmittels oder der Fluoreszenzfarbstoffs die Wellenlänge des durch die Hautoberfläche 10 eingestrahlten Lichts 13 einschließen. Die Emmisionsbande des Fluoreszenzmittels oder des Fluoreszenzfarbstoffs sollte die zur Spektroskopie erforderlichen Wellenlängen einschließen. Z.B. kann die Emission möglichst breitbandig ausgestaltet sein und eine oder mehrere charakteristische Absorptionsbanden des zu messenden Analyten (vollständig oder nahezu oder möglichst vollständig) einschließen.

Gemäß einer Ausführungsform der vorliegenden Erfindung ist der das Fluoreszenzlicht abstrahlende Bereich 113 einer der wie folgt ausgestalteten Bereiche: ein aufgerauter und/oder mit streunenden Partikeln belegter Bereich einer mit der Hautoberfläche verlaufenden Oberfläche des Substrats 111 oder der Vorrichtung 11 oder ein Licht streuender Bereich im Volumen des Substrats 111 oder der Vorrichtung 11. Die auf diese Art ausgestalteten das Fluoreszenzlicht abstrahlenden Bereiche 113 erlauben eine punktförmige oder eine nahezu punktförmige Abstrahlung oder Emission des Fluoreszenzlichts 14, wodurch die oben genannten Eigenschaften der Unterscheidbarkeit des durch die Vorrichtung 11 (bzw. durch den das Fluoreszenzlicht abstrahlenden Bereich 113) emittierten Fluoreszenzlichts 14 und des anderweitig emittierten Lichts 14'. Ferner wird die Zuverlässigkeit der nach der Auswertung des emittierten Fluoreszenzlichts 14 erhaltenen Ergebnisse erhöht.

Die oben genannte positive Wirkung (Unterscheidbarkeit, Zuverlässigkeit) des aufgerauten und/oder mit streunenden Partikeln belegten Bereichs 113 beruht auf Störung der Totalreflexion in dem Bereich 113. Die positive Wirkung wird gemäß einer Ausführungsform dadurch verstärkt, dass die Vorrichtung 11 oder das Substrat 111 einen Reflektor an einer der Hautoberfläche 10 abgewandten Seite des das Fluoreszenzlicht abstrahlenden Bereichs 113 aufweist und/oder der das Fluoreszenzlicht abstrahlende Bereich 113 an einer der Hautoberfläche 10 abgewandten Oberfläche des Substrats 111 oder der Vorrichtung 11 ausgestaltet ist. Durch den Reflektor wird das gestreute Licht zusätzlich in die Richtung der Hautoberfläche 10 gelenkt.

Wenn der das Fluoreszenzlicht abstrahlende Bereich 113 ein Licht streuender Bereich im Volumen des Substrats 111 oder der Vorrichtung 11 ist, dann kann dieser beispielsweise durch mikroskopisch kleine Lufteinschlüsse oder Mikrorisse im Substrat 111 oder in der Vorrichtung 11 ausgeformt sein. Wenn beispielsweise Glas als Material für das Substrat 111 oder die Vorrichtung 11 verwendet wird, können die mikroskopisch kleinen Lufteinschlüsse oder Mikrorisse im Substrat 111 oder in der Vorrichtung 11 durch Anwenden der Laser-Innengravur erzeugt sein. Wenn beispielsweise Kunststoff als Material für das Substrat 111 oder die Vorrichtung 11 verwendet wird, kann der das Fluoreszenzlicht abstrahlende Bereich 113 durch Einbettung eines Streukörpers im Volumen des Substrats 111 oder der Vorrichtung 11 ausgeformt werden.

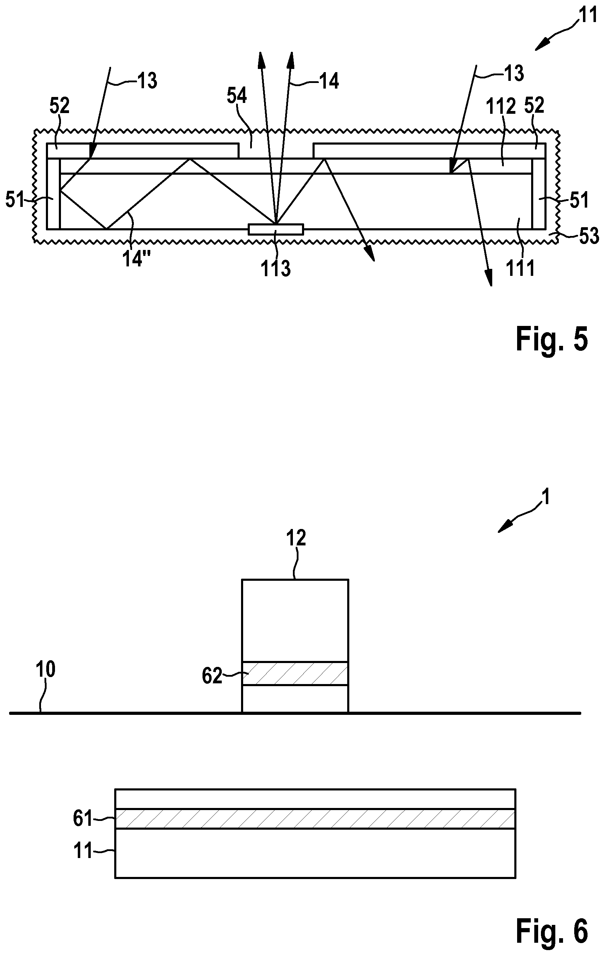

Fig. 5 zeigt eine Ausgestaltung der unter die Hautoberfläche einsetzbaren Vorrichtung 11 gemäß einer weiteren Ausführungsform der vorliegenden Erfindung. Dabei ist anzumerken, dass jede der weiteren durch Fig. 5 gezeigten Komponenten der Vorrichtung 11 einzeln und/und oder in Kombination mit zumindest einer weiteren der in Fig. 5 gezeigten Komponenten zum Ausgestalten der Vorrichtung 11 verwendet werden kann. Fig. 5 zeigt die Vorrichtung 11 mit allen weiteren gemäß der vorliegenden Erfindung möglichen Komponenten, um die Beschreibung kurz und übersichtlich zu halten.

Gemäß der vorliegenden Ausführungsform weist die Vorrichtung 11 einen Spiegel 51 an zumindest einer Stirnseite der Vorrichtung 11 oder an zumindest einer Kantfläche der Vorrichtung 11 respektive auf, wobei der zumindest eine Spiegel 51 ausgestaltet ist, in Schicht 112 oder Substrat 111 propagierendes Fluoreszenzlicht 14" zu reflektieren, welches andernfalls die Vorrichtung 11 an der betreffenden Kante oder Stirnseite verlassen würde, weil es die Voraussetzungen für Totalreflexion nicht erfüllt. Die Stirnseite oder die Kantenfläche der Vorrichtung 11 ist diejenige Seite oder Fläche der Vorrichtung, die nicht (im Wesentlichen parallel) mit der Hautoberfläche 10 verläuft. Vielmehr verläuft sie gegen die Hautoberfläche 10, z.B. im Wesentlichen senkrecht zu der Hautoberfläche 10. Die Vorrichtung 11 weist zumindest einen Spiegel 51 auf. Lichtstrahlen 13, 14", die unter flachen Winkeln zur Ebene der Vorrichtung 11 oder des Substrats 111 laufen und deswegen an der Ober- oder Unterseite der Vorrichtung 11 totalreflektiert werden, treffen unter relativ steilen Winkeln auf die Kantenfläche und können dort auskoppeln. Dieses lässt sich durch eine (z.B. metallische, vorzugsweise silberne oder goldene) Verspiegelung an diesen Flächen unterbinden. Auf diese Weise wird das Hinausstrahlen des in der Vorrichtung 11 eingefangenen Lichts 14" vermieden. Dadurch wird die Menge des anderweitigen vom Gewebe emittierten Lichts 14' reduziert, was schlussendlich zur besseren Unterscheidbarkeit zwischen dem von der Vorrichtung 11 (bzw. von dem das Fluoreszenzlicht abstrahlende Bereich 113) emittierten Licht 14 und dem anderweitigen vom Gewebe aus emittierten Licht 14' führt und bessere Ergebnisse der Auswertung des emittierten Fluoreszenzlichts 14 mit sich bringt.

Gemäß der vorliegenden Ausführungsform weist die Vorrichtung 11 eine das Fluoreszenzlicht steuernde Schicht 52 auf der der Hautoberfläche zugewandten Seite der Vorrichtung 11 auf. Die das Fluoreszenzlicht steuernde Schicht 52 ist oberhalb der fluoreszierenden Schicht 112 ausgeformt. Dabei ist die das Fluoreszenzlicht steuernde Schicht 52 für das auf die Hautoberfläche 10 abgestrahlte Licht 13 im Wesentlichen transparent und für das emittierte Fluoreszenzlicht 14 im Wesentlichen reflektierend oder absorbierend. Die das Fluoreszenzlicht steuernde Schicht ist ausgestaltet, das Abstrahlen des Fluoreszenzlichts 14 an eine bestimmte Position der Hautoberfläche 10 zu leiten. Gemäß der vorliegenden Ausführungsform ist die das Fluoreszenzlicht steuernde Schicht 52 gegenüber dem das Fluoreszenzlicht abstrahlenden Bereich 113 offen (siehe Öffnung 54 in Fig. 5), sodass das durch den das Fluoreszenzlicht abstrahlenden Bereich 113 abstrahlende Fluoreszenzlicht 14 an die bestimmte Position der Hautoberfläche 10 abgestrahlt wird. Dabei wird das Fluoreszenzlicht 14 durch die Öffnung 54 der das Fluoreszenzlicht steuernden Schicht 52 in die Richtung der Hautoberfläche 10 emittiert oder abgestrahlt. Zwar erfolgt die Lichtauskopplung gemäß der vorliegenden Erfindung hauptsächlich an der Streuzone bzw. im das Fluoreszenzlicht abstrahlenden Bereich 113, da das Fluoreszenzlicht 14 jedoch isotrop abgestrahlt wird, unterliegt die Lichtauskopplung nicht vollständig der Totalreflexion. Bei entsprechend steilem Winkel zur Grenzfläche zwischen der Vorrichtung 11 und der Umgebung der Vorrichtung 11 können Lichtstrahlen des durch die fluoreszierende Schicht erzeugten Fluoreszenzlichts 14" die Vorrichtung 11 verlassen. Auch die Streuung an meist unvermeidbaren Materialdeffekten kann zu einer ungewollten Auskopplung des durch die fluoreszierende Schicht erzeugten Fluoreszenzlichts 14" führen. Durch das Verwenden der das Fluoreszenzlicht steuernden Schicht 52 kann die Emission oder Abstrahlung des erzeugten Fluoreszenzlichts 14 vollständig auf die dafür vorgesehene Stelle bzw. auf den das Fluoreszenzlicht abstrahlenden Bereich 113 erreicht werden. Gemäß der vorliegenden Ausführungsform befindet sich die das Fluoreszenzlicht steuernde Schicht 52 zwischen der fluoreszierenden Schicht 112 und der Hautoberfläche 10, z.B. auf der fluoreszierenden Schicht 112. Wenn die Vorrichtung 11 von einem Verkapselungsmaterial, das unten noch genauer erläutert wird, umgeben ist, kann die das Fluoreszenzlicht steuernde Schicht 52 auch auf dessen Oberfläche ausgestaltet sein. Die das Fluoreszenzlicht steuernde Schicht 52 ist oberhalb des das Fluoreszenzlicht abstrahlenden Bereichs 113 geöffnet (siehe Öffnung 54). Darüber hinaus kann die Divergenz des abgestrahlten oder emittierten Fluoreszenzlichts 14 durch das Verhältnis der Radien der Öffnung 54 der das Fluoreszenzlicht steuernden Schicht 52 und des das Fluoreszenzlicht abstrahlenden Bereichs 113 eingestellt werden.

Die das Fluoreszenzlicht steuernde Schicht 52 kann beispielsweise durch dielektrische Interferenzfilter ausgebildet sein. Durch gezieltes Design einer Abfolge dielektrischer Schichten mit verschiedenen Brechzahlen lässt sich das Reflexions- und Transmissionsverhalten solcher Schichtsysteme nahezu beliebig einstellen. Dabei sind entsprechende Hoch-Tief- oder Bandpass-Filter für alle Spektralbereiche verfügbar und einsetzbar.

Ferner kann die das Fluoreszenzlicht steuernde Schicht 52 kann beispielsweise durch transparente leitfähige Schichten ausgebildet sein. Dabei kann die das Fluoreszenzlicht steuernde Schicht 52 beispielsweise aus transparent leitfähigen Metalloxiden (TCO-Materialien) wie z.B. verschieden dotierten Indium-Zink- oder Zinkoxiden ausgestaltet sein. Diese weisen die Eigenschaft der Transparenz auf, zeichnen sich durch gute Transmissionseigenschaften im sichtbaren Spektralbereich aus und sind gleichzeitig im Nahinfrarot gute Reflektoren. Für die Lage der Reflektionskante ist dann die Plasmafrequenz wichtig.

Darüber hinaus kann die das Fluoreszenzlicht steuernde Schicht 52 mittels Farbstoffsysteme ausgebildet sein. Hierzu ist jeder Farbstoff (organisch, anorganisch oder Quantendots) geeignet, dessen Absorptionsbande die Emissionsbande des im Implantat verwendeten Fluoreszenzfarbstoffs aber nicht die Wellenlänge des Anregungslichts einschließt. Ferner sollte der Farbstoff nach der Bestrahlung über die Hautoberfläche 10 strahlungslos in den Grundzustand zurückkehren oder in einem für die Spektroskopie irrelevanten Spektralbereich emittierten, wobei dieser Spektralbereich nicht mit dem Emissions-Spektralbereich des durch die Vorrichtung 11 (bzw. durch den das Fluoreszenzlicht abstrahlenden Bereich 113) emittierten Fluoreszenzlichts 14 überlappt. Die vorstehend beschriebene das Fluoreszenzlicht steuernde Schicht 52 kann auch als spektral selektive Schicht (SSS) bezeichnet werden.

Gemäß der vorliegenden Ausführungsform ist die Vorrichtung 11 in einem Low-n-Material 53 eingekapselt oder verkapselt. Es ist schwierig, ein aus verschiedenen Materialien bestehendes Bauteil biokompatibel zu gestaltet. Bezüglich der Vorrichtung 11 sollte dieses jedoch vorgenommen werden, da die Vorrichtung 11 zum Implantieren unter die Hautoberfläche 10 vorgesehen ist und somit biokompatibel sein sollte. D.h. der Organismus und sein Gewebe darf nicht durch die Vorrichtung 11 geschädigt werden. Ferner ist die für die optische Funktionalität entscheidende Ober- und Grenzflächenqualität über längere Zeit kaum aufrecht zu erhalten. So wird ein lebender Organismus die meisten Fremdkörper binnen kurzer Zeit elektromechanisch schädigen oder zumindest mit einem Proteinfilm bedecken. Beides beeinflusst die Grenzflächenreflektionen. Eine Lösung dieses Problems ist die Einkapselung der Vorrichtung 11 in ein Material 53, das keine physiologische Schädigung hervorruft und resistent gegen biochemische Angriffe ist. Entscheidend für die Optik ist die Qualität der geschützten inneren Grenzflächen. Die Qualität der Kapseloberfläche ist irrelevant und kann durchaus rau sein, da die Umgebung der Vorrichtung 11 (d.h. das Gewebe) ohnehin sehr stark streut. Die Brechzahl bzw. der Brechindex des Kapselmaterials 53 sollte möglichst nah an der Brechzahl bzw. an den Brechindex des umgebenden Gewebes liegen, um die optische Wirkung der Grenzfläche zu minimieren. Ist dies nicht möglich, so sollte die Brechzahl des Kapselmaterials 53 eher kleiner sein als die des umliegenden Gewebes.

Gemäß einer weiteren Ausführungsform der vorliegenden Erfindung kann anstelle der das Fluoreszenzlicht steuernden Schicht 52 oder der spektral selektiven Schicht (SSS) 52 auch ein Kapselmaterial 53 gewählt werden, das das Fluoreszenzlicht 14 absorbiert. D.h. bei geeignetem Kapselmaterial 53 kann das Ausgestalten der Vorrichtung 11 mit der das Fluoreszenzlicht steuernden Schicht 52 auch ausgelassen werden. In diesem Fall muss an oder über dem das Fluoreszenzlicht abstrahlenden Bereich 113 dennoch Transparenz hergestellt werden. Dieses kann beispielsweise durch eine lokal dünnere oder anders zusammengesetzte Kapselschicht über dem das Fluoreszenzlicht abstrahlenden Bereich 113 erreicht werden. Diese lokal anders ausgestaltete Kapselschicht würde die gleiche Platzierung wie die Stelle der Öffnung 54 in der das Fluoreszenzlicht steuernden Schicht 52 haben.

Fig. 6 zeigt eine Anordnung 1 gemäß einer Ausführungsform der vorliegenden Erfindung. Die Anordnung 1 der Fig. 6 entspricht der Anordnung 1 der Fig. 1 mit dem Unterschied dass die Vorrichtung 11 ein die Vorrichtung 11 ausrichtendes Element 61 aufweist, mittels dessen die Vorrichtung 11 unter der Hautoberfläche 10 nichtinvasiv ausgerichtet werden kann. Die Vorrichtung 11 kann allgemein wie in der vorliegenden Anmeldung dargestellt ausgestaltet sein.

Das die Vorrichtung 11 ausrichtendes Element 61 kann beispielsweise ein ferromagnetisches Element sein. Es kann beispielsweise eine Platte unter dem transparenten Material 53, ein die Vorrichtung 11 umschließender Ring oder eine Art "Trog", der das transparente Material 53 von allen Seiten (außer der der Hautoberfläche 10 zugewandten Seite) umschließt, sein. Durch Anlegen eines Magnetfelds (zum Beispiel über Elektro- oder Permanentmagneten an der Licht detektierenden Vorrichtung 12) kann nichtinvasiv eine Kraft auf die Vorrichtung 11 ausgeübt werden, um die Vorrichtung 11 in die Richtung der auf der Hautoberfläche 10 befindlichen Licht detektierenden Vorrichtung 12 zu ziehen und/oder hinsichtlich der Licht detektierenden Vorrichtung 12 auszurichten. Trägt das ferromagnetische Element 61 selbst ein permanentes magnetisches Moment, können auch nichtinvasiv Drehmomente vermittelt werden.

Gemäß einer Ausführungsform der vorliegenden Erfindung kann durch nichtinvasive Vermittlung von Kräften und/oder Drehmomenten unter Verwendung des ausrichtenden Elements 61 eine eindeutige Positionierung der Vorrichtung 11 zu der Licht detektierenden Vorrichtung 12 sichergestellt werden.

Gemäß einer Ausführungsform der vorliegenden Erfindung kann unter Verwendung des ausrichtenden Elements 61 die Vorrichtung 11 zu der Licht detektierenden Vorrichtung 12 hingezogen werden, wodurch das zwischen den beiden Vorrichtungen 11, 12 liegende Gewebe komprimiert wird und wodurch der Anteil von Blut und Gewebeflüssigkeit in dem dazwischen liegendem Gewebe verringert wird. Durch ein periodisches Anziehen und Loslassen der Vorrichtung 11 an oder von der Licht detektierenden Vorrichtung 12 kann das dazwischen liegende Gewebe alternierend mit verschiedenen Flüssigkeitsanteilen gemessen werden. Dieses ist insbesondere dann nützlich, um Einflüsse von Gewebe und Flüssigkeiten auf das Absorptionsspektrum zu trennen.

Gemäß einer Ausführungsform der vorliegenden Erfindung kann man unter Verwendung des ausrichtenden Elements 61 die Vorrichtung 11 vor der optischen Messung (kurz) vibrieren lassen. Dadurch können lokale Diffusionsprozesse beschleunigt und chemische Gradienten in der unmittelbaren Nachbarschaft der Vorrichtung 11 homogenisiert werden. Periodisches Anziehen und Abstoßen der Vorrichtung 11 bezüglich der auf der Hautoberfläche 10 aufgesetzten Licht detektierenden Vorrichtung 12 spült das dazwischenliegende Gewebe durch periodisches "fluten" und "auspressen" der Flüssigkeiten im Gewebe.

Gemäß Fig. 6 weist die Licht detektierende Vorrichtung 12 ein ausrichtendes Element 62 aufweist, das ausgestaltet ist, die Vorrichtung 11 unter der Hautoberfläche nichtinvasiv derart auszurichten, dass eine das Licht abstrahlende und das Fluoreszenzlicht empfangende Seite der Licht detektierende Vorrichtung 12 im Wesentlichen oberhalb der das Licht absorbierenden und das Fluoreszenzlicht abstrahlenden Seite der Vorrichtung 11 ist.

Die vorliegende Erfindung erlaubt auch weitere Ausführungsformen, in denen die Schichten, Bereiche der Vorrichtung 11, die für das Sammeln des eingestrahlten Lichts 13 und für das Emittieren des Fluoreszenzlichts 14 zuständig sind, geometrisch getrennt sind. Auf diese Weise wird eine räumliche Trennung der beiden Lichtintensitäten erreicht, so dass bei einer entsprechenden Ausgestaltung der Licht detektierenden Vorrichtung 12 kein vom Gewebe anderweitig zurückgestreutes Licht 14' in die Licht detektierende Vorrichtung 12 gelangen kann bzw. von der Licht detektierenden Vorrichtung 12 empfangen wird.

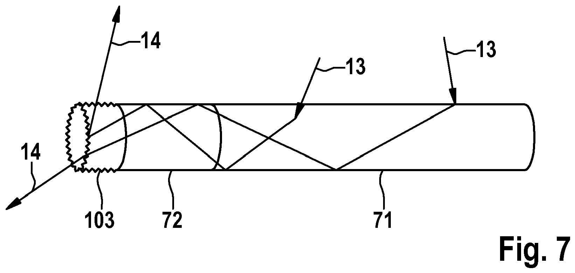

Fig. 7 zeigt eine Ausgestaltung der unter die Hautoberfläche einsetzbaren Vorrichtung 11 gemäß einer Ausführungsform der vorliegenden Erfindung. Die Vorrichtung 11 und somit auch ihr Substrat 111 besteht gemäß der vorliegenden Ausführungsform aus einer Glasfaser (oder aus einem anderen Lichtleiter), deren ein Abschnitt 71 mit einer fluoreszierenden Schicht 112 versehen ist und die unter Verwendung der fluoreszierenden Schicht 112 das durch die Hautoberfläche 10 durchstrahlende Licht 13 sammelt. Alternativ kann der Abschnitt 71 auch homogen mit dem Fluoreszenzmittel eingefärbt sein und es kann auf eine Schicht verzichtet werden. Gemäß der vorliegenden Ausführungsform schließt sich dem fluoreszierenden Abschnitt 71 ein weiterer Abschnitt 72 ohne die fluoreszierende Schicht 112 an, wobei in dieser Ausführungsform der weitere Abschnitt 72 optional ist. Am Ende der Faser 11 tritt das Fluoreszenzlicht 14 aus. Dieses Ende kann als der oben beschriebene das Fluoreszenzlicht abstrahlende Bereich 113 gesehen werden. Gemäß der vorliegenden Ausführungsform weist das Endstück oder der das Fluoreszenzlicht abstrahlende Bereich 113 eine raue Oberfläche auf, um das Fluoreszenzlicht 14 verstärkt weg von der Faserachse (in die Richtung der Hautoberfläche 10 und in die Richtung der Licht detektierenden Vorrichtung 12) abzustrahlen oder zu emittieren. Diese Ausführungsform der Vorrichtung 11 kann beispielsweise gut in einem schwach streuendem Gewebe eingesetzt werden, da die gemäß der vorliegenden Ausführungsform ausgestaltete Vorrichtung ermöglicht, den Anteil ballistischer Photonen, die ohne Streuung in gerader Linie durch das Gewebe zur Hautoberfläche 10 gelangen, an der Hautoberfläche 10 zu erhöhen. Die höhere Konzentration des emittierten Fluoreszenzlichts 14 ergibt sich durch eine deutlich größere Länge des mit der fluoreszierenden Schicht 112 behafteten, Licht sammelnden Faserteils 71 gegenüber dem auskoppelnden Endstück 113.

Die vorstehend erläuterten Ausführungsformen mit den dort erläuterten spezifischen Aspekten sind mit einander kombinierbar. Die vorliegende Erfindung ermöglicht das verschiedenartiges Kombinieren der oben beschriebenen Schichten der erfindungsgemäßen Lichtquelle 11. Diese Kombinationen wurden aufgrund deren Vielzahl und zwecks einer präzisen Darstellung der vorliegenden Erfindung nicht vollständig aufgeführt, sind dem Fachmann aber im Lichte der beschriebenen und beanspruchten Erfindung ersichtlich. Durch die vorliegende Erfindung wie beschrieben und beansprucht wird ein implantierbarer Fluoreszenzkonzentrator als subkutane Lichtquelle für die optische Absorptionsspektroskopie oberflächennaher Gewebeschichten in-vivo eingesetzt. Dadurch können sichere und zuverlässige Ergebnisse der optischen Absorptionsspektroskopie erreicht werden. Ferner können verschiedene Analyten mit unterschiedlichen Absorptionseigenschaften sicher und zuverlässig quantifiziert werden. Dabei betrifft die vorliegende Erfindung die als Lichtquelle ausgestaltete Vorrichtung, eine Licht detektierende Vorrichtung, die das durch die Lichtquelle ausgestrahlte Licht zwecks optischer Absorptionsspektroskopie detektiert, und eine Anordnung, welche die als Lichtquelle ausgestaltete Vorrichtung und die Licht detektierende Vorrichtung aufweist.

## Patentansprüche

1. Vorrichtung (11), die ausgestaltet ist, unter eine Hautoberfläche (10) eingesetzt zu werden; eine fluoreszierende Schicht (112) und ein Substrat (111) mit einem das Fluoreszenzlicht (14) abstrahlenden Bereich (113), aufweist, wobei die fluoreszierende Schicht (112) ausgestaltet ist, ein auf die Hautoberfläche (10) abgestrahltes Licht (13) zu absorbieren und das absorbierte Licht in ein Fluoreszenzlicht (14) zu wandeln; wobei die fluoreszierende Schicht (112) auf dem Substrat (111) ausgebildet ist, das aus einem Material mit einem Brechungsindex größer als ein Brechungsindex eines die Vorrichtung (11) umgebenden Gewebes besteht, um das Fluoreszenzlicht (14) aufzunehmen und mittels Totalreflexion dem das Fluoreszenzlicht (14) abstrahlenden Bereich (113) zuzuleiten, der ausgestaltet ist, das Fluoreszenzlicht (14) aufzunehmen und in die Richtung der Hautoberfläche (10) abzustrahlen.

2. Vorrichtung (11) nach Anspruch 1, wobei die fluoreszierende Schicht (112) eine der wie folgt ausgestalteten Schichten ist: eine Schicht aus einem fluoreszierenden Farbstoff oder eine Schicht, die aus dem Material des Substrats (111) und aus einem fluoreszierenden Farbstoff ausgestaltet ist.

3. Vorrichtung (11) nach einem der vorstehenden Ansprüche, wobei der das Fluoreszenzlicht abstrahlende Bereich (113) kleiner als die der Hautoberfläche (10) zugewandte Seite der Vorrichtung (11) ist; und/oder der das Fluoreszenzlicht abstrahlende Bereich (113) einer der wie folgt ausgestalteten Bereiche ist: ein aufgerauter und/oder mit streunenden Partikeln belegter Bereich einer mit der Hautoberfläche (10) verlaufenden Oberfläche des Substrats (111) oder ein Licht streuender Bereich im Volumen des Substrats (111).

4. Vorrichtung (11) nach Anspruch 3, wobei, wenn der das Fluoreszenzlicht abstrahlende Bereich (113) ein aufgerauter und/oder mit streunenden Partikeln belegter Bereich ist, die Vorrichtung (11) einen Reflektor an einer der Hautoberfläche abgewandten Seite des das Fluoreszenzlicht abstrahlenden Bereichs (113) aufweist und/oder der das Fluoreszenzlicht abstrahlende Bereich (113) an einer der Hautoberfläche (10) abgewandten Oberfläche des Substrats (111) ausgestaltet ist.

5. Vorrichtung (11) nach einem der vorstehenden Ansprüche, wobei die Vorrichtung (1) zumindest einen Spiegel (51) an zumindest einer Stirnseite der Vorrichtung (11) aufweist, wobei der zumindest eine Spiegel (51) ausgestaltet ist, in die Vorrichtung (11) hinein strahlende und gegen die Stirnseite der Vorrichtung (11) strahlende Lichtstrahlen des Fluoreszenzlichts in die Vorrichtung (11) zu reflektieren.

6. Vorrichtung (11) nach einem der vorstehenden Ansprüche, wobei die Vorrichtung (11) eine das Fluoreszenzlicht steuernde Schicht (52) auf der der Hautoberfläche (10) zugewandten Seite der Vorrichtung (11) aufweist, wobei die das Fluoreszenzlicht steuernde Schicht (52) oberhalb der fluoreszierenden Schicht (112) ausgeformt ist, wobei die das Fluoreszenzlicht steuernde Schicht (52) für das auf die Hautoberfläche abgestrahlte Licht (13) im Wesentlichen transparent ist, wobei die das Fluoreszenzlicht steuernde Schicht (52) für das Fluoreszenzlicht (14) im Wesentlichen reflektierend oder absorbierend ist und wobei die das Fluoreszenzlicht steuernde Schicht (52) ausgestaltet ist, das Abstrahlen des Fluoreszenzlichts (14) an eine bestimmte Position der Hautoberfläche zu leiten.

7. Vorrichtung (11) nach Anspruch 6 und nach einem der Ansprüche 4 bis 5, wobei die das Fluoreszenzlicht steuernde Schicht (52) gegenüber dem das Fluoreszenzlicht abstrahlenden Bereich (113) offen (54) ist, sodass das durch den das Fluoreszenzlicht abstrahlenden Bereich (113) abstrahlende Fluoreszenzlicht (14) an die bestimmte Position der Hautoberfläche abgestrahlt wird.

8. Vorrichtung (11) nach einem der vorstehenden Ansprüche, wobei die Vorrichtung (11) ein die Vorrichtung ausrichtendes Element (61) aufweist, mittels dessen die Vorrichtung (11) unter der Hautoberfläche (10) nichtinvasiv ausgerichtet werden kann.

9. Anordnung (1), die eine Vorrichtung (11) nach einem des vorstehenden Ansprüche 1 bis 8 und eine Licht detektierende Vorrichtung (12) aufweist, wobei die Licht detektierende Vorrichtung (12) ausgestaltet ist, Licht (13) auf die Hautoberfläche (10) abzustrahlen, und das Fluoreszenzlicht (14), das von der Vorrichtung (11) abgestrahlt wurde, zu detektieren und zu empfangen.

10. Anordnung (1) nach Anspruch 9, wobei die Licht detektierende Vorrichtung (12) ein Filterelement (2) aufweist, welches das Fluoreszenzlicht (14) zum Auswerten in die Licht detektierende Vorrichtung (12) durchlässt und ein anderweitiges von der Hautoberfläche emittiertes Licht blockiert.

11. Anordnung (1) nach Anspruch 9 oder 10, wobei die Licht detektierende Vorrichtung (12) ein ausrichtendes Element (62) aufweist, das ausgestaltet ist, die Vorrichtung (11) unter der Hautoberfläche (10) nichtinvasiv derart auszurichten, dass eine das Licht abstrahlende und das Fluoreszenzlicht empfangende Seite der Licht detektierenden Vorrichtung (12) im Wesentlichen oberhalb der das Licht absorbierenden und das Fluoreszenzlicht abstrahlenden Seite der Vorrichtung (11) ist.

## Claims

1. Device (11), which is configured to be inserted under a skin surface (10); has a fluorescent layer (112) and a substrate (111) having a fluorescent-light-emitting (14) region (113), wherein the fluorescent layer (112) is configured to absorb light (13) that is emitted onto the skin surface (10) and to convert the absorbed light into fluorescent light (14); wherein the fluorescent layer (112) is formed on the substrate (111) which consists of a material having a refractive index that is greater than a refractive index of a tissue surrounding the device (11) in order to receive the fluorescent light (14) and to guide it, by way of total internal reflection, to the region (113) emitting the fluorescent light (14), which region is configured to receive the fluorescent light (14) and to emit it in the direction of the skin surface (10).

2. Device (11) according to Claim 1, wherein the fluorescent layer (112) is one of the layers configured as follows: a layer made of a fluorescent dye or a layer configured from the material of the substrate (111) and from a fluorescent dye.

3. Device (11) according to either of the preceding claims, wherein the fluorescent-light-emitting region (113) is smaller than the side of the device (11) facing the skin surface (10); and/or the fluorescent-light-emitting region (113) is one of the regions configured as follows: a roughened region, and/or a region covered with scattering particles, of a surface of the substrate (111) that extends with the skin surface (10), or a light-scattering region in the volume of the substrate (111).

4. Device (11) according to Claim 3, wherein, if the fluorescent-light-emitting region (113) is a roughened region and/or a region covered with scattering particles, the device (11) has a reflector at a side of the fluorescent-light-emitting region (113) facing away from the skin surface and/or the fluorescent-light-emitting region (113) is formed on a surface of the substrate (111) facing away from the skin surface (10).

5. Device (11) according to one of the preceding claims, wherein the device (1) has at least one mirror (51) at least at one front end of the device (11), wherein the at least one mirror (51) is configured to reflect light rays of the fluorescent light, which radiate into the device (11) and against the front end of the device (11), into the device (11).

6. Device (11) according to one of the preceding claims, wherein the device (11) has a fluorescent-light-controlling layer (52) on the side of the device (11) which faces the skin surface (10), wherein the fluorescent-light-controlling layer (52) is formed above the fluorescent layer (112), wherein the fluorescent-light-controlling layer (52) is substantially transparent for the light (13) emitted onto the skin surface, wherein the fluorescent-light-controlling layer (52) is substantially reflective or absorbing for the fluorescent light (14), and wherein the fluorescent-light-controlling layer (52) is configured to direct the emission of the fluorescent light (14) to a specific position on the skin surface.

7. Device (11) according to Claim 6 and according to one of Claims 4 to 5, wherein the fluorescent-light-controlling layer (52) is open (54) with respect to the fluorescent-light-emitting region (113) such that the fluorescent light (14) emitted by the fluorescent-light-emitting region (113) is emitted to a specific position on the skin surface.

8. Device (11) according to one of the preceding claims, wherein the device (11) has an element (61) which aligns the device, with which the device (11) can be aligned noninvasively under the skin surface (10).

9. Arrangement (1) having a device (11) according to one of the preceding Claims 1 to 8 and a light-detecting device (12), wherein the light-detecting device (12) is configured to emit light (13) onto the skin surface (10), and to detect and receive the fluorescent light (14) which was emitted by the device (11).

10. Arrangement (1) according to Claim 9, wherein the light-detecting device (12) has a filter element (2) which transmits the fluorescent light (14) for evaluation in the light-detecting device (12) and blocks other light emitted by the skin surface.

11. Arrangement (1) according to Claim 9 or 10, wherein the light-detecting device (12) has an aligning element (62) which is configured to noninvasively align the device (11) under the skin surface (10) such that a side of the light-detecting device (12) which emits the light and receives the fluorescent light is substantially above the side of the device (11) which absorbs the light and emits the fluorescent light.

## Revendications

1. Dispositif (11), lequel est configuré en vue d'être utilisé sous une surface de la peau (10) et lequel présente une couche fluorescente (112) et un substrat (111) avec une zone de rayonnement (113) de la lumière fluorescente (14) ;
dans lequel la couche fluorescente (112) est configurée en vue d'absorber de la lumière diffusée (13) sur la surface de la peau (10) et en vue de transformer la lumière absorbée en une lumière fluorescente (14) ; dans lequel la couche fluorescente (112) est formée sur le substrat (111), lequel est constitué d'un matériau qui présente un indice de réfraction supérieur à un indice de réfraction d'un tissu entourant le dispositif (11), en vue de capter la lumière fluorescente (14) et de canaliser la zone de rayonnement (113) de la lumière fluorescente (14) au moyen de la réflexion totale, laquelle zone de rayonnement est configurée en vue de capter la lumière fluorescente (14) et d'émettre des rayonnements en direction de la surface de la peau (10).

2. Dispositif (11) selon la revendication 1, dans lequel la couche fluorescente (112) est l'une des couches configurées comme suit : une couche qui est composée d'un colorant fluorescent ou une couche qui est configurée à partir du matériau du substrat (111) et à partir d'un colorant fluorescent.

3. Dispositif (11) selon l'une des revendications précédentes, dans lequel la zone de rayonnement (113) de la lumière fluorescente est plus petite que la face du dispositif (11) qui est orientée vers la surface de la peau (10) ; et/ou la zone de rayonnement (113) de la lumière fluorescente est l'une des zones configurées comme suit : une zone d'une surface du substrat (111) qui s'étend avec la surface de la peau (10), laquelle zone est rugueuse et/ou enduite de particules errantes, ou une zone dans le volume du substrat (111), laquelle disperse la lumière.

4. Dispositif (11) selon la revendication 3, dans lequel, quand la zone de rayonnement (113) de la lumière fluorescente est une zone rugueuse et/ou enduite de particules errantes, le dispositif (11) présente un réflecteur au niveau d'une face de la zone de rayonnement (113) de la lumière fluorescente, laquelle face se trouve à l'opposé de la surface de la peau, et/ou la zone de rayonnement (113) de la lumière fluorescente est configurée au niveau d'une surface du substrat (111), laquelle se trouve à l'opposé de la surface de la peau (10).

5. Dispositif (11) selon l'une des revendications précédentes, dans lequel le dispositif (1) présente tout au moins un miroir (51) au niveau de tout au moins une face frontale du dispositif (11), dans lequel le tout au moins un miroir (51) est configuré en vue de réfléchir dans le dispositif (11) les rayons lumineux de la lumière fluorescente qui sont diffusés contre la face frontale du dispositif (11) et qui sont diffusés à l'intérieur du dispositif (11).

6. Dispositif (11) selon l'une des revendications précédentes, dans lequel le dispositif (11) présente une couche (52) qui commande la lumière fluorescente et qui se trouve sur la face du dispositif (11) qui est orientée vers la surface de la peau (10) ;
dans lequel la couche (52) qui commande la lumière fluorescente est formée au-dessus de la couche fluorescente (112), dans lequel la couche (52) qui commande la lumière fluorescente est, pour l'essentiel, transparente pour la lumière (13) qui est diffusée sur la surface de la peau dans lequel la couche (52) qui commande la lumière fluorescente (14) est, pour l'essentiel, réfléchissante ou absorbante pour la lumière fluorescente (14) et
dans lequel la couche (52) qui commande la lumière fluorescente est configurée en vue de diriger le rayonnement de la lumière fluorescente (14) au niveau d'une certaine position déterminée de la surface de la peau.

7. Dispositif (11) selon la revendication 6 et selon l'une des revendications 4 à 5, dans lequel la couche (52) qui commande la lumière fluorescente est ouverte (54) vis-à-vis de la zone de rayonnement (113) de la lumière fluorescente, de telle sorte que la lumière fluorescente (14) diffusée par l'intermédiaire de la zone de rayonnement (113) de la lumière fluorescente est diffusée au niveau de la position déterminée de la surface de la peau.

8. Dispositif (11) selon l'une des revendications précédentes, dans lequel le dispositif (11) présente un élément (61) permettant de guider le dispositif, au moyen duquel le dispositif (11) peut être dirigé de manière non invasive sous la surface de la peau (10).

9. Agencement (1) qui présente un dispositif (11) selon l'une des revendications précédentes 1 à 8 ainsi qu'un dispositif (12) détectant la lumière, dans lequel le dispositif (12) qui détecte la lumière est configuré en vue de diffuser de la lumière (13) sur la surface de la peau (10), ainsi qu'en vue de détecter et de recevoir la lumière fluorescente (14) qui a été diffusée par le dispositif (11).

10. Agencement (1) selon la revendication 9, dans lequel le dispositif (12) qui détecte la lumière présente un élément de filtrage (2), lequel laisse passer la lumière fluorescente (14) dans le dispositif (12) détectant la lumière à des fins d'analyse, et lequel empêche la pénétration de tout autre type de lumière émise par la surface de la peau.

11. Agencement (1) selon la revendication 9 ou 10, dans lequel le dispositif (12) qui détecte la lumière présente un élément (62) de guidage, lequel est configuré en vue de guider le dispositif (11) de manière non invasive sous la surface de la peau (10), de telle sorte qu'une face du dispositif (12) détectant la lumière, laquelle face diffuse la lumière et reçoit la lumière fluorescente, se trouve, pour l'essentiel, au-dessus de la face du dispositif (11) qui absorbe la lumière et qui diffuse la lumière fluorescente.
